(19) Europäisches Patentamt
European Patent Office
Office européen des brevets

(11) Veröffentlichungsnummer: **0 330 080 A2**

(12) **EUROPÄISCHE PATENTANMELDUNG**

(21) Anmeldenummer: **89102714.6**

(51) Int. Cl.[4]: **G01N 33/68 , G01N 33/543**

(22) Anmeldetag: **17.02.89**

(30) Priorität: **22.02.88 DE 3805447**

(43) Veröffentlichungstag der Anmeldung: **30.08.89 Patentblatt 89/35**

(84) Benannte Vertragsstaaten: **AT BE CH DE ES FR GB GR IT LI LU NL SE**

(71) Anmelder: **Drenckhahn, Detlef, Prof. Dr.**
**Ulmenweg 30**
**D-3550 Marburg(DE)**

(72) Erfinder: **Drenckhahn, Detlef, Prof.Dr.**
**Ulmenweg 30**
**D-3550 Marburg(DE)**

(74) Vertreter: **Görtz, Dr. Fuchs, Dr. Luderschmidt**
**Patentanwälte**
**Sonnenberger Strasse 100 Postfach 26 26**
**D-6200 Wiesbaden(DE)**

# (54) Verfahren und Mittel zur Bestimmung von Nierenschäden.

(57) Es sind ein Verfahren sowie ein Mittel zur quantitativen Bestimmung von Nierenschäden beschrieben, wobei poly- oder monoklonale Antikörper gegen die Proteine Villin und/oder Fimbrin eingesetzt werden, die im Urin erscheinen, sobald Beeinträchtigungen der Niere, beispielsweise Abstoßungsreaktionen bei einer transplantierten Niere oder Tubulusentzündungen, auftreten.

EP 0 330 080 A2

# Verfahren und Mittel zur Bestimmung von Nierenschäden

Die Erfindung betrifft ein Verfahren zur Bestimmung von Nierenschäden, bei dem poly- oder monoklonale Antikörper mit in der Niere auftretenden Proteinen in Berührung gebracht werden und das erhaltene Reaktionsprodukt anschließend einem immunologischen Nachweisverfahren unterzogen wird, sowie ein Mittel, mit dem dieses Verfahren durchgeführt werden kann.

Nierenschäden machen sich üblicherweise zuerst durch Schädigungen des Parenschyms bemerkbar, die beispielsweise bei fortlaufender Einnahme von nierenschädigenden Medikamenten, wie Phenacetin, bei einer gesunden Niere oder bei Nichteinnahme eines Immunosuppresivums bei einer transplantierten Niere auftreten. Insofern ist die Früherkennung von Parenschymschäden der Niere ein wichtiges medizinisches Problem.

Derzeit werden im wesentlichen drei Untersuchungsverfahren angewandt, um Parenchymschäden der Niere zu diagnostizieren :

a. Das klassische Verfahren besteht darin, daß durch Biopsie Gewebsproben aus der Niere entnommen werden. Dieses invasive Verfahren ist jedoch mit Komplikationen behaftet und kommt nur in Ausnahmefällen zum Einsatz.

b. Es wird die Aktivität von Gewebsenzymen im Urin bestimmt. Dieser klinische Test ist jedoch in seiner Aussagefähigkeit stark eingeschränkt, weil durch ihn keine verläßlich Lokalisation des Schädigungsortes in der Niere erfolgen kann und die Aktivität der Enzyme im Urin durch zahlreiche äußere Faktoren beeinflußt wird. Dieser Test ist bereits in qualitativer Hinsicht zu unspezifisch.

c. Desweiteren wurde versucht, auf immunologische Weise Schädigungen der Niere nachzuweisen. Dabei wurden monoklonale Antikörper gegen unbekannte oder unzureichend charakterisierte Nierenproteine eingesetzt, die in erhöhten Mengen im Urin ausgeschieden werden. Diese Methode kann allenfalls als grobes qualitatives Verfahren zur Bestimmung von Nierenschädigungen angesehen werden, da die Proteine, gegen die die Antikörper gerichtet sind, nicht isoliert und deshalb nicht zur quantitativen Kalibrierung der Meßwerte herangezogen werden. Insofern sind mit dieser Methods keinesfalls quantitative Aussagen möglich, die zur Abschätzung des Schweregrades einer Nierenschädigung aber erforderlich sind.

Der Erfindung liegt daher die Aufgabe zugrunde, das Verfahren der eingangs erwähnten Art so zu verbessern, daß auch geringe bzw. im Anschwellen befindliche Nierenschädigungen praktisch quantitativ erfaßt werden können.

Der Erfindung liegt weiterhin die Aufgabe zugrunde, ein Mittel zur Durchführung des Verfahrens zur Verfügung zu stellen.

Die Lösung der Aufgabe erfolgt dadurch, daß man als Antikörper solche einsetzt, die gegen die Proteine Fimbrin oder Villin spezifisch sind.

Überraschenderweise wurde nunmehr festgestellt, daß die Proteine Villin und Fimbrin, die zwei molekulardefinierte Proteine des proximalen Nierentubulusepithels sind, in praktisch quantitativer Weise als Maßstab für eine Nierenschädigung herangezogen werden können. Dabei können diese Proteine im Urin quantitative ermittelt werden. Beide Proteine werden nämlich vermehrt bei Schädigung der Niere ausgeschieden, die den proximalen Tubulus betreffen oder ihn einbeziehen. Somit steht erfindungsgemäß ein nichtinvasives, diagnostisches Verfahren zur Verfügung, mit dem Schädigungen der Niere, beispielsweise verursacht durch Durchblutungsstörungen, drohendes akutes Nierenversagen oder drohende Tranplantatabstoßung, frühzeitig quantitativ erfaßt werden können.

Mit demn erfindungsgemäßen Verfahren können Aussagen zu folgenden klinischen Fragestellungen gemacht werden, nämlich zur

a. Lokalisation einer Nierenschädigung im proximalen Tubulus,

b. Beurteilung des Schweregrades der Zellschädigung,

c. Ansprechbarkeit der Nierenschädigung auf therapeutische Maßnahmen und

d. Abschätzung des Risikos der Behandlung von Nierenkranken und nierentransplantierten Patienten mit Medikamenten, die nierenschädigende Nebenwirkungen haben.

Die Proteine Villin und Fimbrin lassen sich einfach im Urin nachweisen, mit dem sie ausgeschieden werden. Üblicherweise sind nur etwa 0,1 ml Urin notwendig, das der Patient ausscheidet. Insofern hat das Nachweisverfahren keine schädigenden oder toxischen Wirkungen auf den Patienten, da die Untersuchung im ausgeschiedenen Urin in vitro erfolgt.

Die Proteine Villin und Fimbrin lassen sich in reiner Form darstellen. Insofern ist eine exakte Festlegung der Meßwerte möglich, d.h. die Proteine Fimbrin und Villin lassen sich exakt quantitativ im Urin bestimmen, so daß hierdurch ein Parameter für den Schweregrad der Schädigung der Nierentubuli Verfügung steht. Bei dem erfindungsgemäßen Verfahren wird vorteilhaft eine Kalibrierung des eingesetzten Reagenz durchgeführt, um den Gehalt der Proteine Villin und/oder Fimbrin im Urin quantitativ festzulegen.

Ein typisches, einfaches Verfahren, mit dem die Proteine Villin und Fimbrin im Urin quantitative nachgewiesen werden können, besteht aus folgenden Merkmalen : Die Proteine Villin und Fimbrin werden auf an sich bekannte Weise aus dem Darmepithel des Huhns oder des Schweins isoliert. Dieses Verfahren ist von A. Bretscher und K. Weber in Proc. Natl. Acad. Sci. USA 76 (1979), S. 2321-2325 oder J. Cell. Biol. 86 (1980) Seite 335-340 oder D. Drenckhahn et al. in Cell. Tiss. Res. 228 (1983) beschrieben. Auf die darin erläuterten Isolierungsverfahren wird ausdrücklich Bezug genommen.

Auf gleiche Weise können diese Proteine aus dem Darmepithel von chirurgisch entfernten Darmstücken des Menschen isoliert werden.

Antikörper gegen Fimbrin und/oder Villin können nach den vorstehend angegebenen Literaturstellen oder aber auch nach allgemein bekannten Methoden hergestellt werden. Solche Methoden sind beispielsweise in der Monographie von R.J. Mayer und J.H. Walker mit dem Titel "Immunochemical methods in the biological sciences: Enzymes und proteins" erschienen in Academic Press, London (1980), beschrieben.

So werden z.B. die isolierten Proteine in Mengen von 50-100 µg mit und ohne Freund'schem kompletten oder inkompletten Adjuvanz Kaninchen oder Meerschweinchen unter die Rückenhaut oder in die Bauchhöhe injiziert. In dreiwöchigen Abständen wird die gleiche Menge an Proteinen wieder injiziert. Nach der dritten oder späteren Injektion werden gute Titer polyklonaler Antikörper erhalten, die nach üblichen Standardverfahren affinitätsgereinigt und auf ihre Spezifität getestet werden. Ein solches Verfahren wurde in den o.a. Publikationen von Drenckhahn et al., 1983, oder Bretscher und Weber, 1979, 1980 oder von Talian et al. in J. Cell. Biol. 97 (1983), S. 1277-1282 beschrieben, worauf Bezug genommen wird.

Neben polyklonalen Antikörpern lassen sich auch monoklonale Antikörper einsetzen, die in Mäusen und Ratten nach ebenfalls allgemein bekannten Verfahren hergestellt werden (vgl. die Publikation von J.H. Peters et al., "Monoklonale Antikörper: Herstellung und Charakterisierung", erschienen in Springer-Verlag, Berlin (1985).

Derartige monoklonale Antikörper gegen Villin werden bereits zur histologischen Diagnose von Tumoren des Darms, in dem ebenfalls Villin freigesetzt wird, kommerziell von der Firma Dianova, Hamburg, angeboten.

Erfindungsgemäß werden die poly- oder monoklonalen Antikörper gegen die Proteine Villin und/oder Fimbrin an der Oberfläche von Reaktionsträgern gebunden. Unter "Bindung" ist dabei entweder eine physikalische Adsorption der Antikörper an ein Substrat oder aber eine chemische kovalente Bindung dieser Antikörper an das Substrat zu verstehen.

Als Reaktionsträger können Filtermaterialien, Materialien von Reaktionsgefäßen, wie Mikrotiterplatten, Säulenchromatographiematerialien, wie Sepharose und dergl. in Frage kommen. Die Antikörper werden in wässeriger Lösung, vorteilhafterweise in einer wässerigen Pufferlösung mit dem Substratmaterial so lange in Berührung gebracht, bis sich ein Adsorptionsgleichgewicht gebildet hat. Diese Gleichgewichtseinstellung ist temperaturabhängig und dauert bei etwa 21 °C ca. 3 Stunden und bei 4 °C ca. 12 Stunden.

Als Substratmaterial ist ein Kunststoffmaterial, insbesondere Polystyrol, bevorzugt, das üblicherweise zur Herstellung von Mikrotiterplatten eingesetzt wird.

Nach Abschluß der Bindungsreaktion werden die adsorbierten Antikörper zur Absättigung freier Proteinbindungsstellen mit Proteinlösungen, beispielsweise Albuminlösungen, Gelatinelösungen oder Magermilch ggf. unter Zusatz von Kochsalz und/oder Detergens behandelt.

Anschließend werden die so behandelten Antikörper-Beschichtungen mehrfach gewaschen, woraufhin die Behandlung mit einer Urinprobe erfolgen kann.

Die Inkubation mit Urin wird so lange durchgeführt, bis sich ein Reaktionsgleichgewicht zwischen den Antikörpern und den Proteinen Villin und/oder Fimbrin eingestellt hat. Vorteilhafterweise dauert der Inkubationsvorgang etwa 30-90 Minuten. Anschließend entfernt man den Urin und füllt, sofern Mikrotiterplatten eingesetzt werden, erneut die Löcher mit der vorstehend angegebenen Waschlösung, die in bestimmten Zeitabständen mehrfach gewechselt wird.

Der so erzeugte Antikörper-Protein-Bindungskomplex wird anschließend einer immunologischen Quantifizierungsreaktion in einem zweiten Verfahrensschritt unterzogen.

Zur Durchführung dieser im zweiten Verfahrensschritt stattfindenden Quantifizierungsreaktion wird der am Substrat haftende Antikörper-Protein-Bindungskomplex erneut mit Antikörpern gegen Villin oder Fimbrin behandelt.

Sofern im ersten Verfahrensschritt ein polyklonaler Antikörper verwendet wurde, wird im zweiten Verfahrensschritt entweder der gleiche polyklonale Antikörper oder aber ein monoklonaler Antikörper gegen Villin oder Fimbrin eingesetzt.

Wird jedoch gemäß einer zweiten Ausführungsform ein monoklonaler Antikörper im ersten Verfahrensschritt eingesetzt, dann kann als Antikörper im zweiten Verfahrensschritt entweder ein polyklonaler oder ein vom ersten Antikörper unterschiedlicher zweiter monoklonaler Antikörper verwendet werden, der gegen eine andere Bindungsstelle des Proteinmoleküls gerichtet ist.

Nachstehend wird der in diesem zweiten Verfahrensschritt eingesetzte Antikörper als "zweiter Antikörper" bezeichnet.

Erfindungsgemäß werden die Löcher der Mikrotiterplatten mit einer diesen zweiten Antikörper enthaltenden Lösung ebenfalls ca. 30-90 Minuten gefüllt und inkubiert. Anschließend wird die zweite Antikörperlösung entfernt, woraufhin die Löcher mit einer Waschlösung gespült werden. Es bleibt auf dem Substrat zurück ein Komplex, bestehend aus dem ersten Antikörper, den gebundenen Proteinen Villin oder Fimbrin und dem zweiten Antikörper, nachstehend als "Quantifizierungskomplex" bezeichnet.

Dieser Quantifizierungskomplex kann auf mehrfache Weise einer Quantifizierungsreaktion unterzogen werden :

Gemäß einer ersten Ausführungsform wird der zweite Antikörper einer kovalenten Kopplung mit Enzymen, beispielsweise Peroxidase, alkalischer Phosphatase, Glukoseoxidase oder Betagalaktosidase unterworfen. Die Menge der im Quantifizierungskomplex gebundenen enzymmarkierten zweiten Antikörper kann anschließend durch eine Enzymfarbreaktion quantitativ bestimmt werden. Derartige Quantifizierungsreaktionen sind an sich bekannt, wobei einsetzbare Enzyme und typische Enzymfarbreaktionen von L.A. Sternberger in "Immunocytochemistry", Dritte Ausgabe, Verlag John Wiley & Sons, New York (1986) oder von W.D. Kuhlmann in der Monographie "Immunoenzyme techniques in cytochemistry", Verlag Chemie, Weinheim (1984) beschrieben worden sind. Hierauf wird Bezug genommen.

Gemäß einer zweiten Ausführungsform können die zweiten Antikörper nach ansich bekannten Verfahren radioaktiv markiert werden, beispielsweise mit Jod 125. Für diese Zwecke werden radioaktive Markierungskits und Substanzen eingesetzt, die beispielsweise von der Firma NEN, Dreieich, geliefert werden.

Die Menge der gebundenen zweiten Antikörper kann dabei durch einen Teilchenzähler bzw. Strahlenzähler direkt quantitativ ermittelt werden.

In einer dritten Ausführungsform der Quantifizierungsreaktion kann der Komplex mit einem dritten Antikörper behandelt werden, der gegen den gebundenen zweiten Antikörper spezifisch ist. Für diese Zwecke können beispielsweise Antikörper gegen Mäuseimmunoglobuline oder Kaninchenimmunoglobuline eingesetzt werden. Solche Antikörper sind in markierter Form mit Enzymen oder Radioisotopen kommerziell erhältlich und werden beispielsweise von den Firmen Dianova, Hamburg; Janssen, Belgien oder NEN, Dreieich, vertrieben. Anschließend erfolgt der bereits oben erwähnte Quantifizierungsschritt, wobei entweder die Farbreaktion quantitativ ausgewertet oder aber die radioaktive Strahlung bestimmt werden.

Diese dritten Antikörper können auch in nichtmarkierter Form als Brückenantikörper dienen, welche Enzym-Anti-Enzymimmunkomplexe bilden, die ggf. in einem vierten Inkubationsschritt eingebracht werden. Diese indirekten Immunglobulinnachweisverfahren sind ebenfalls aus der Literatur bekannt, beispielsweise in der vorstehend erwähnten Monographie von Sternberger, worauf Bezug genommen wird. Im übrigen sind Enzym-Anti-Enzymimmunkomplexe ebenfalls kommerziell erhältlich und werden beispielsweise von Firmen Dianova, Hamburg oder Janssen, Belgien, vertrieben.

In einer vierten Quantifizierungsreaktion, die nachstehend detailliert beschrieben ist und insofern bevorzugt ist, wird die Quantifizierung von Villin oder Fimbrin im Urin dadurch durchgeführt, daß die zweiten Antikörper mit Biotin kovalent gekoppelt werden. Die Kopplung von Biotin erfolgt an den zweiten Antikörper nach ansich bekannten Verfahren, die beispielsweise von N.H. Heggeness et al. in J. Cell. Biol. 73 (1977), S. 783-788 beschrieben ist, worauf Bezug genommen wird. Hierzu wird zum Beispiel Biotin-N-hydroxysuccinimid-ester eingesetzt, das von der Firma Calbiochem. Hoechst vertrieben wird.

Die im Quantifizierungskomplex gebundenen biotinilierten zweiten Antikörper werden durch das biotinbindende Protein Streptavidin quantifiziert. Streptavidin ist mit dem Enzym Peroxidase oder den anderen oben erwähnten Enzymen kovalent an den zweiten Antikörper gebunden. Derartige Enzym-Streptavidin-Lösungen sind kommerziell erhältlich und werden beispielsweise von der Firma Calbiochem-Hoechst vertrieben.

Der erhaltene Streptavidin-Enzym-Antikörperkomplex wird anschließend, sofern das Enzym eine Peroxidase ist, mit 1,2-Phenylendiamin und $H_2O_2$-Lösung versetzt, wobei durch die Reaktion von Peroxidase mit Wasserstoffperoxid das farblose Phenylendiamin in einen gelben Farbstoff umgewandelt wird. Der entstandene Farbstoff kann durch seine Extinktion bei 492 nm gegenüber seiner Referenzwellenlänge von 620 nm im UV-Photometer bestimmt werden. Dabei korreliert die Extinktion mit der Menge der gebundenen Villin- und/oder Fimbrin-Moleküle, wie dies nachstehend gezeigt werden kann.

Gemäß einer fünften Ausführungsform können Villin und Fimbrin im Urin auch durch einen Verdrängungsassay nachgewiesen werden. Bei diesem Testverfahren werden Reaktionsträger mit gereinigtem Villin und/oder Fimbrin beschichtet. Anschließend wird ein Gemisch von einer zu untersuchenden Urinprobe und von Antikörpern beigefügt, die gegen Villin oder Fimbrin spezifisch sind. Je höher die Menge von Villin oder Fimbrin im Urin ist, desto geringer wird die Menge an Antikörpern sein, die an diejenigen Villin- oder Fimbrinmoleküle binden, die bereits am Reaktionsträger adsorbiert worden waren. Die Menge der gebundenen Antikörper kann anschließend durch direkte Markierung der Antikörper mit Enzymen oder Radioisotopen oder durch die übrigen beschriebenen Ver fahren quantifiziert werden.

Es sei nochmals erwähnt, daß die Quantifizierungsreaktionen, mit denen der Quantifizie rungskomplex behandelt wird, ansich bekannt sind. Insofern sind auch weitere Abwandlungen der Quantifizierungsreaktionen denkbar, sofern die gebundenen Villin oder Fimbrin-Moleküle hierdurch quantitativ erfaßt werden können.

Erfindungsgemäß wird auch ein Mittel zur Durchführung des vorstehend beschriebenen Verfahrens zur Verfügung gestellt.

Gemäß einer ersten Variante A besteht das Mittel aus mono- oder polyklonalen Antikörpern gegen Fimbrin und/oder Villin.

Vorzugsweise werden bereits an einen Reaktionsträger gebundene Antikörper in den Handel gebracht, wobei das Einbringen von Antikörpern in Mikrotiterplatten bevorzugt ist. So kann eine Handelseinheit aus einer Mikrotiterplatte mit einer Vielzahl von Löchern bestehen, wobei jedes Loch mit einer bestimmten Menge Antikörper gegen Villin und/oder Fimbrin versehen ist.

Zusätzlich zu dieser Substrateinheit weist das erfindungsgemäße Mittel vorteilhaft den vorstehend erwähnten zweiten Antikörper zur Herstellung des Quanitfizierungskomplexes auf. An diesen zweiten Antikörper sind vorteilhafterweise Biotingruppen kovalent gebunden. Diese biotinilierten Antikörper können entweder bereits als in fertiger gepufferter Gebrauchslösung in den Handel gebracht werden oder aber zusammen mit der Puffersubstanz in lyophilisierter Form vertrieben werden, wobei das die lyophilisierten Produkte enthaltene Gefäß für den Einsatz lediglich mit einer bestimmten Menge Wasser gefüllt werden muß.

Eine derartige Handelseinheit kann weiterhin - sofern nicht im Labor vorhanden - Konzentrate der vorstehend erwähnten Waschlösungen sowie die Quantifizierungsreagenzien Streptavidin-Peroxidase, $H_2O_2$, Phenylendiamin enthalten, die zur Quantifizierung des Quantifizierungskomplexes eingesetzt werden.

Außerdem werden vorteilhafterweise zur Eichung der Meßwerte mit jeder Substrateinheit die isolierten gereinigten proteine Fimbrin und/oder Villin geliefert, vorteilhafterweise in einer Menge von etwa 4 µg. Diese werden in lyophilisierter Form in einem Gefäß vertrieben, das mit einer bestimmten Menge Wasser oder Kontrollurin vom Verbraucher aufgefüllt wird.

Gemäß einer zweiten Varianten B, die auf dem vorstehend beschriebenen Verdrängungsassay beruht, besteht das Mittel neben den vorstehend unter Variante A genannten Antikörpern gegen Fimbrin und/oder Villin aus den isolierten Proteinen Villin oder Fimbrin.

Die Proteine werden an Reaktionsträger gebunden in den Handel gebracht. Eine Handelseinheit kann aus einer Mikrotiterplatte bestehen, wobei jedes Loch mit einer bestimmten Menge (beispielsweise 0,5 - 1 µg) der isolierten Proteine Villin und/oder Fimbrin versehen ist.

Zusätzlich zu dieser Substrateinheit weist das zweite Mittel die vorstehend erwähnten mono- oder polyklonalen Antikörper gegen Villin oder Fimbrin auf, an die vorteilhafterweise Biotingruppen kovalent gebunden sind.

Diese Antikörper werden entweder als fertige Gebrauchslösung oder in lyophilisierter Form vertrieben, wie in der ersten Ausführungsform beschrieben.

Außerdem enthält vorteilhafterweise jede Einheit dieser Ausführungsform zu Eichzwecken noch die isolierten Proteine Villin und/oder Fimbrin, wie ebenfalls zur Variante A beschrieben.

Dieser Nachweiskit kann weiterhin, wie in der Variante A beschrieben, die vorstehend erwähnten Waschlösungen sowie die Quantifizierungsreagenzien enthalten.

Das nachfolgende Beispiel erläutert die Erfindung :

Polyklonale Antikörper gegen Fimbrin und/oder Villin werden gemäß dem Verfahren von Bretscher und Weber 1979, 1980 und Drenckhahn et al.,1983 hergestellt. Monoklonale Antikörper werden entsprechend dem Verfahren von Peter et al., 1985 hergestellt oder es wird ein monoklonaler Antikörper gegen Villin von der Firma Dianova, Hamburg, eingesetzt.

Diese Antikörper werden an Polystyrol-Mikrotiterplatten, bezogen von der Firma Nunc, Wiesbaden, entsprechend dem Verfahren von Peter et al., 1985, gebunden.

Dazu werden die isolierten Antikörper in einer Immunoglobulin-Konzentration von 5 µg/ml in 0,2 M- Carbonatpuffer mit einem pH-Wert von 10,6 gelöst. Nicht gereinigtes Immunserum wird zweckmäßgerweise in einem Verhältnis von 1:100 im Carbonatpuffer verdünnt. Nachstehend wird der Begriff "Antikörper" als übergreifender Begriff für isolierte Immunglobuline und Immunserum verwendet.

Zur ausreichenden Bindung der Antikörper an die Wand Löcher der Mikrotiterplatten werden jeweils 100 µl der verdünnten Antikörper-Lösung etwa drei Stunden bei 21 °C bzw. zwölf Stunden bei 4 °C in den Löchern der Mikrotiterplatten belassen. Danach wird die Antikörperlösung aus den Löchern entfernt, wobei anschließend die Löcher zur Absättigung freier Bindungsstellen mit etwa 400 µl einer 1 gew.-%igen Gelatinelösung in dem vorstehend angegebenen Karbonatpuffer gefüllt werden.

Nach einer Stunde Inkubationszeit wird die Gelatinelösung wieder entfernt und die Löcher werden mit etwa 400 ul Waschpuffer gefüllt.

Dieser Waschpuffer besteht aus einer mit Phosphat gepufferten Kochsalzlösung (25 mM Phosphatpuffer, 100mM NaCl mit einem pH-Wert von 7,4), der nachstehend PPK genannt wird. Dieser Pufferlösung werden 0,05 Gew.-% des Detergens Tween 20 zugesetzt, das von der Firma Serva, Heidelberg, bezogen werden kann.

Der Waschpuffer wird in 5-minütigen Abständen 3 mal gewechselt.

Die so präparierte Mikrotiterplatte kann entweder unmittelbar der Reaktion mit einer zu untersuchenden Urinprobe ausgesetzt werden oder aber getrocknet und anschließend zum Einsatz verpackt werden.

Die Löcher der so hergestellten Mikrotiterplatte werden jeweils mit 100 µl unverdünntem zu untersuchenden Urin gefüllt, wobei der Urin anschließend etwa 1,5 Stunden in den Löchern belassen wird. Danach wird der Urin entfernt und die Löcher werden erneut mit 400 µl des vorstehend angegebenen Waschpuffers inkubiert, der in 5-minütigen Abständen 3 mal gewechselt wird.

Nach Entfernung des Waschpuffers werden die Löcher wiederum jeweils mit 100 µl der für die Beschichtung der Platten angegebenen polyklonalen Antikörper gegen Villin und/oder Fimbrin inkubiert. An diese zweiten Antikörper wird zunächst nach der Methode von Heggeness, 1977 (siehe oben) Biotin kovalent gekoppelt. Die biotinilierten Antikörper werden in einer Immunglobulinkonzentration von 10 µg/ml in einem Puffer gelöst, der aus 150 mM NaCl, 20 mM Tris-HCl, 0,5 mM Harnstoff und 1% Gelatine besteht und einen pH-Wert von 7,4 aufweist.

Diese Antikörperlösung wird wiederum etwa 90 Minuten in den Löchern belassen. Nach Entfernung der zweiten Antikörperlösung aus den Löchern werden die Löcher jeweils mit 400 ml PPK mit 0,05 Gew.-% Tween 20 gefüllt, das in 5-minütigen Abständen drei mal gewechselt wird.

Hierauf wird die Quantifizierungsreaktion auf folgende Weise durchgeführt.

Nach Entfernung des Waschpuffers werden die Löcher mit 100 µl der o.a. phosphat-gepufferten Kochsalzlösung (PPK, pH 7,4) gefüllt. Diese Lösung enthält das biotinbindende Protein Streptavidin, das mit dem Enzym Peroxidase kovalent gekoppelt ist.

Das Peroxidase-Streptavidin wird als Stammlösung von der Firma Calbiochem-Hoechst bezogen und in einer Verdünnung mit 1:5.000 in PPK, pH 7,4 eingesetzt. Man beläßt diese Streptavidin-Lösung etwa 1 Stunde bei 21 °C in den Löchern und entfernt diese anschließend.

Danach werden die Löcher mit etwa 400 µl des vorstehend angegebenen Waschpuffers (PPk, pH 7,4, 0,05 Gew.-% Tween 20) gefüllt. Diese Pufferlösung wird in 5-minütigen Abständen drei mal gewechselt.

Anschließend werden die Löcher jeweils mit 100 µl eines Phosphat-Zitratpuffers (0,2 M Dinatriumhydrogenphosphat, 0,1 M Zitronensäuremonohydrat mit einem pH von 5) gefüllt, der je 1 ml Puffer 1,5 mg 1,2-Phenylendiamin (bezogen von der Firma Merck) und 1 µl einer 30%igen $H_2O_2$-Lösung (unter der Bezeichnung "Perhydrol" von der Firma Merck vertrieben) enthält.

Das Phenylendiamin und $H_2O_2$ werden unmittelbar vor Gebrauch dem Phosphat-Zitratpuffer zugegeben. Anschließend werden mit dieser Lösung die Löcher 30 Minuten inkubiert.

Durch Zusatz von 50 µl 0,5 N Schwefelsäure/Loch wird die Reaktion beendet.

Proportional zur Menge der gebundenen biotinilierten Antikörper und der an die Biotingruppen gebundenen, mit Peroxidase markierten Streptavidinmoleküle entsteht durch Oxidation des farblosen Phenylendiamins ein gelber Farbstoff, dessen Extinktion bei 492 nm gegenüber der Referenzwellenlänge von 620 nm im UV-Photometer gemessen wird.

In Fig. 1 sind zwei Standardeichkurven von Villin und von Fimbrin gezeigt, aus denen die Korrelation zwischen der Extinktion einerseits und der Konzentration des Proteins andererseits grafisch dargestellt ist.

Diese Eichkurve wurde dadurch erstellt, daß definierte Mengen Villin (a) oder Fimbrin(b) dem Urin einer gesun den Normalperson zugegeben und mit Hilfe des vorstehend erwähnten immunologischen Verfahrens quantifiziert wurden. Dabei enthält das Mikrotiterloch 1 : 10 µg/ml, 2 : 5 µg/ml, 3 : 2,5 µg/ml, 4 : 1,25 µg/ml, 5 : 0,625 µg/ml und 6 : 0,312 µg/ml Protein.

Aus diesen Standardkurven ist ersichtlich, daß sowohl Villin als auch Fimbrin quantitativ durch die vorstehende UV-photometrische Bestimmung im Urin ermittelt werden können.

In Fig. 2 ist grafisch eine Fallstudie an einem Patienten mit transplantierter Niere über 30 Tage (Abszisse) festgehalten, wobei je Tag der Villingehalt in den Urinproben des Patienten bestimmt wurde.

Aus Fig. 2 ist ersichtlich, daß der Patient eine Villinkonzentration mit einer Basislinie der Extinktion von 0,05 aufweist. Dabei können die absoluten Konzentrationen von Villin im Urin des Patienten aus der Eichkruve gemäß Fig. 1 abgelesen werden.

Die in Fig. 2 eingezeichneten Pfeile weisen auf Abstoßungskrisen hin, die klinisch durch erhöhte Kreatininwerte und klinische Symtomatik verifiziert wurden. Die Therapie dieser Abstoßungskrisen bestand in erhöhten Dosen von Immunsuppressiva, was sich in einem Abfall der Villinausscheidungen im Urin, d.h. im Zurückgehen der Schädigungsgefahr der Niere widerspiegelte.

## Ansprüche

1. Verfahren zur Bestimmung von Nierenschäden, bei dem poly- oder monoklonale Antikörper mit in der Niere auftretenden Proteinen in Berührung gebracht und das erhaltene Reaktionsprodukt anschließend einer immunologischen Reaktion unterzogen wird, dadurch gekennzeichnet, daß man als Antikörper solche einsetzt, die gegen die Proteine Fimbrin oder Villin spezifisch sind.

2. Verfahren nach Anspruch 1, dadurch gekennzeichnet, daß man die Antikörper gegen Fimbrin oder Villin entweder nach Variante A an die Oberfläche eines Substrats bindet, wobei anschließend die gebundenen Antikörper mit einer zu untersuchenden Urinprobe unter Bildung eines quantifizierbaren Protein-Antikörper-Komplexes in Berührung gebracht werden oder nach Variante B (Verdrängungsassay) in der Urinprobe vorgelegt und die erhaltene Lösung mit an die Oberfläche eines Substrats gebundenem Villin oder Fimbrin in Berührung bringt, wobei die Antikörper mengenmäßig umgekehrt proportional zu der im Urin enthaltenen Menge an Villin oder Fimbrin an die Substratoberfläche binden, und anschließend jeweils das erhaltene Reaktionsprodukt der immunologischen Reaktion unterzieht.

3. Verfahren nach Anspruch 2, dadurch gekennzeichnet, daß man nach Variante A gemäß Anspruch 2 eine die Antikörper enthaltende Lösung mit der Substratoberfläche in Berührung bringt, nach Einstellung des Adsorptionsgleichgewichts die Lösung entfernt, das beschichtete Substrat mit einer Waschlösung spült, danach die Inkubation des Substrats mit einer Urinprobe durchführt, nach Entfernen der Urinprobe erneut die Substratoberfläche mit Waschlösung spült und schließlich die an die Antikörper gebundenen Proteine Villin oder Fimbrin einer immunologischen Quantifizierungsreaktion unterzieht.

4. Verfahren nach Anspruch 3, dadurch gekennzeichnet, daß man die immunologische Quantifizierungsreaktion mit einem zweiten Antikörper durchführt, wobei man bei Einsatz eines ersten polyklonalen Antikörpers den gleichen polyklonalen oder einen monoklonalen Antikörper gegen Villin oder Fimbrin als zweiten Antikörper einsetzt oder bei Einsatz eines ersten monoklonalen Antikörpers einen polyklonalen Antikörper oder einen vom ersten Antikörper unterschiedlichen zweiten monoklonalen Antikörper gegen Villin oder Fimbrin verwendet sowie die verwendeten zweiten Antikörper mit Peroxidase, Glucoseoxidase, alkalischer Phosphatase, Beta-Galaktosidase oder mit anderen Enzymen koppelt.

5. Verfahren nach Anspruch 3 oder 4, dadurch gekennzeichnet, daß die in Anspruch 3 beanspruchte immunologische Quantifizierungsreaktion dadurch erfolgt, daß man die nach Anspruch 4 gekoppelten zweiten Antikörper mit den an die ersten Antikörper gebundenen Proteine Villin oder Fimbrin in Berührung bringt, anschließend nach Einstellung des Bindungsgleichgewichts die Lösung entfernt, das beschichtete Substrat mit einer Waschlösung spült und nachfolgend in einer spezifischen Enzym-Substratreaktion die Menge des gebildeten Substrats mit optischen oder radiochemischen Methoden bestimmt.

6. Verfahren nach Anspruch 4 oder 5, dadurch gekennzeichnet, daß die immunologische Quantifizierungsreaktion dadurch erfolgt, daß die Menge der gebundenen zweiten Antikörper durch einen dritten Antikörper ermittelt wird, der gegen den zweiten, nicht jedoch den ersten Antikörper gerichtet ist, wobei die dritten Antikörper mit Enzymen oder Radioisotopen markiert sind und ihre Menge durch Enzym-Substratreaktionen oder radiochemische Methoden quantifiziert wird oder die Menge der gebundenen dritten Antikörper durch einen Enzym-Anti-Enzymkomplex quantifiziert wird, mit der die an die zweiten Antikörper gebundenen dritten Antikörper in Berührung gebracht werden.

7. Verfahren nach Anspruch 2, dadurch gekennzeichnet, daß man gemäß Variante B die die Proteine Villin oder Fimbrin enthaltende Lösung mit der Substratoberfläche in Berührung bringt, nach Einstellung des Adsorptionsgleichgewichtes die Lösung entfernt, das beschichtete Substrat mit einer Waschlösung spült, danach die Inkubation des Substrates mit der Urinprobe durchführt, in der eine definierte Menge monoklonaler oder polyklonaler Antikörper gegen Villin oder Fimbrin vorliegt, nach Einstellung eines Gleichgewichtes zwischen den an Fimbrin oder Villin im Urin gebundenen Antikörpern und den an das Substrat gebundenen Antikörpern die Urinprobe entfernt, die Substratoberfläche mit einer Waschlösung spült und anschließend die an das Substrat gebundenen Antikörper dem Quantifizierungsverfahren unterzieht.

8. Verfahren nach Anspruch 7, dadurch gekennzeichnet, daß das Quantifizierungsverfahren durch Kopplung der Antikörper mit den in Anspruch 4 beanspruchten Enzymen oder Radioisotopen und anschließender enzymatischer oder radiochemischer Nachweismethode erfolgt oder daß die gebundenen Antikörper mit Biotin gekoppelt werden und die biotinilierten Antikörper durch das Protein Avidin quantifiziert werden, an das Enzyme oder Radioisotope gekoppelt sind oder daß die Quantifizierungsreaktion dadurch erfolgt, daß die Menge der gebundenen Antikörper durch einen zweiten Antikörper ermittelt wird, der gegen den gebundenen Antikörper gerichtet ist, wobei vorzugsweise die zweiten Antikörper mit Enzymen oder Radioisotopen markiert sind und ihre Menge durch Enzym-Substratreaktion oder radiochemische Methoden quantifiziert wird und vorzugsweise die Menge der gebundenen zweiten Antikörper durch einen Enzym-Anti-Enzymkomplex quantifiziert wird.

9. Mittel zur Bestimmung von Nierenschäden, enthaltend polyklonale oder monoklonale Antikörper gegen Fimbrin oder polyklonale Antikörper gegen Villin.

10. Verwendung von monoklonalen Antikörpern gegen Villin zum Nachweis der Schädigung der Niere.

11. Ausführungsform nach Anspruch 9 oder 10, dadurch gekennzeichnet, daß die Antikörper an ein Substrat gebunden sind, das vorzugsweise ein Kunststoffmaterial, insbesondere eine Mikrotiterplatte aus Polystyrol, ist.

12. Ausführungsform nach Anspruch 9 oder 10, dadurch gekennzeichnet, daß die Antikörper zur Zugabe zur zu untersuchenden Urinprobe in Lösung oder in lyophilisierter Form vorliegen und Villin oder Fimbrin an das Substrat gemäß Anspruch 11 gebunden sind, wobei die Proteine Villin oder Fimbrin zusätzlich als Eichsubstanzen zugesetzt sind.

13. Ausführungsform nach einem der Ansprüche 9 bis 12, gekennzeichnet durch einen Gehalt an einem zweiten Antikörper, der gleich oder unterschiedlich von den in Anspruch 9 oder 10 beanspruchten Antikörpern sein kann und mit Biotin markiert ist, wobei der zweite Antikörper in Lösung oder in lyophilisierter Form vorliegt.

14. Ausführungsform nach einem der Ansprüche 9 bis 13, gekennzeichnet durch ein immunologisches Reagenz zum Nachweis der nach Verfahren 4, 5 oder 7 gebundenen Antikörper, wobei vorzugsweise das Reagenz Peroxidase-konjugiertes Streptavidin, 1,2-Phenylendiamin und $H_2O_2$ aufweist.

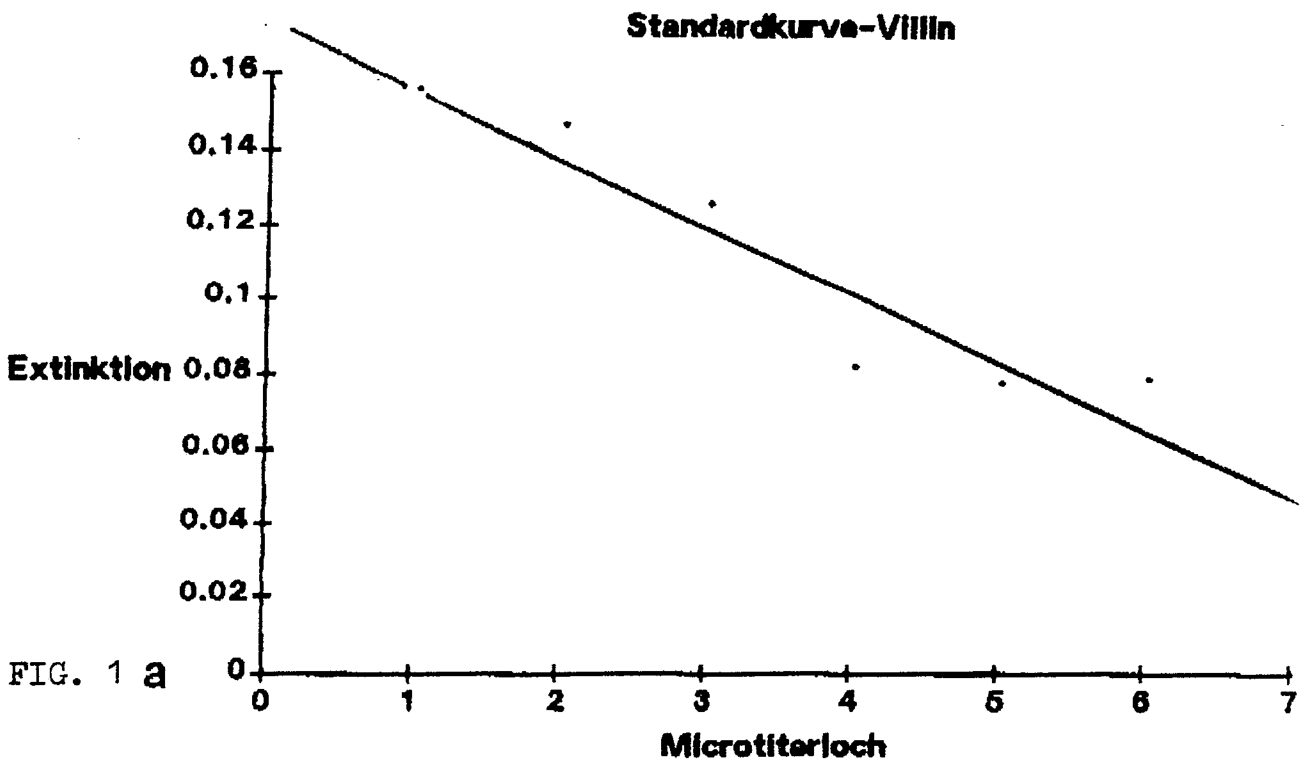
Standardkurve-Villin
Extinktion
0.16
0.14
0.12
0.1
0.08
0.06
0.04
0.02
0
0
1
2
3
4
5
6
7
Microtiterloch

FIG. 1 a

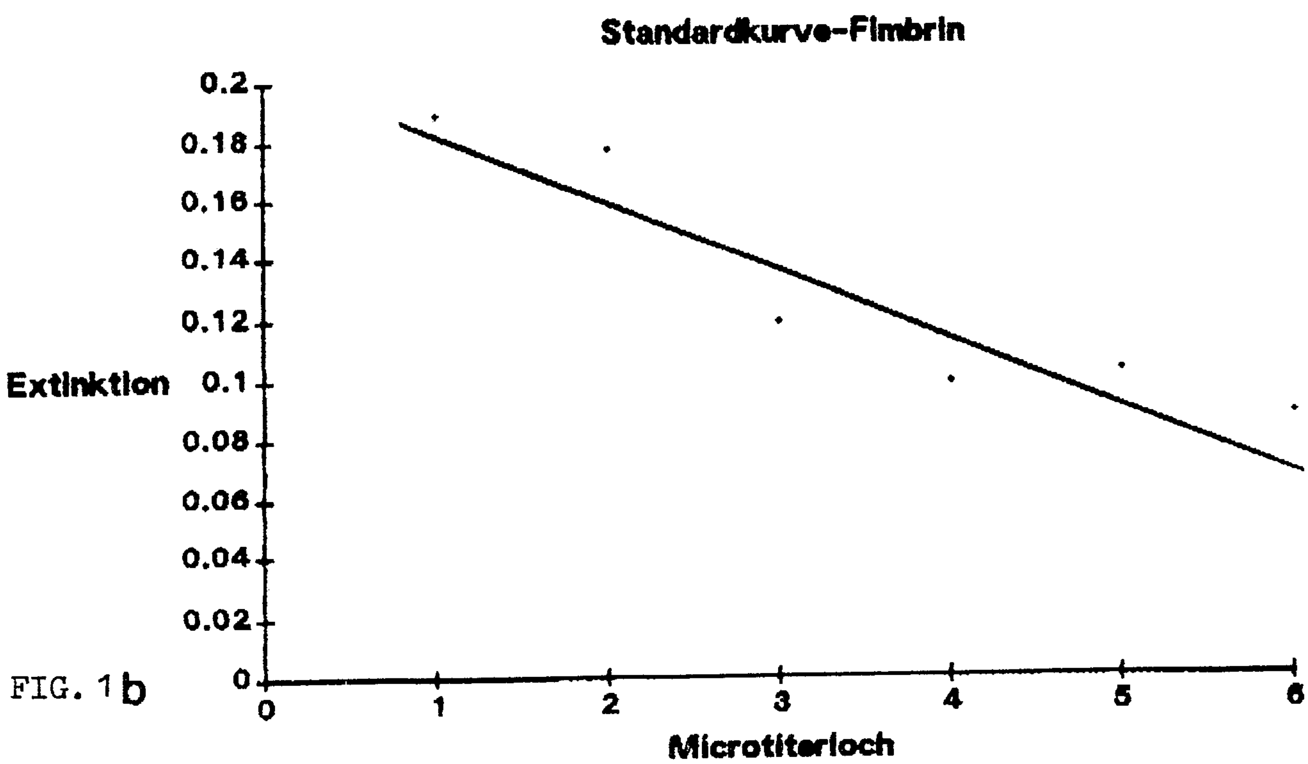
Standardkurve-Fimbrin
Extinktion
0.2
0.18
0.16
0.14
0.12
0.1
0.08
0.06
0.04
0.02
0
0
1
2
3
4
5
6
Microtiterloch

FIG. 1 b

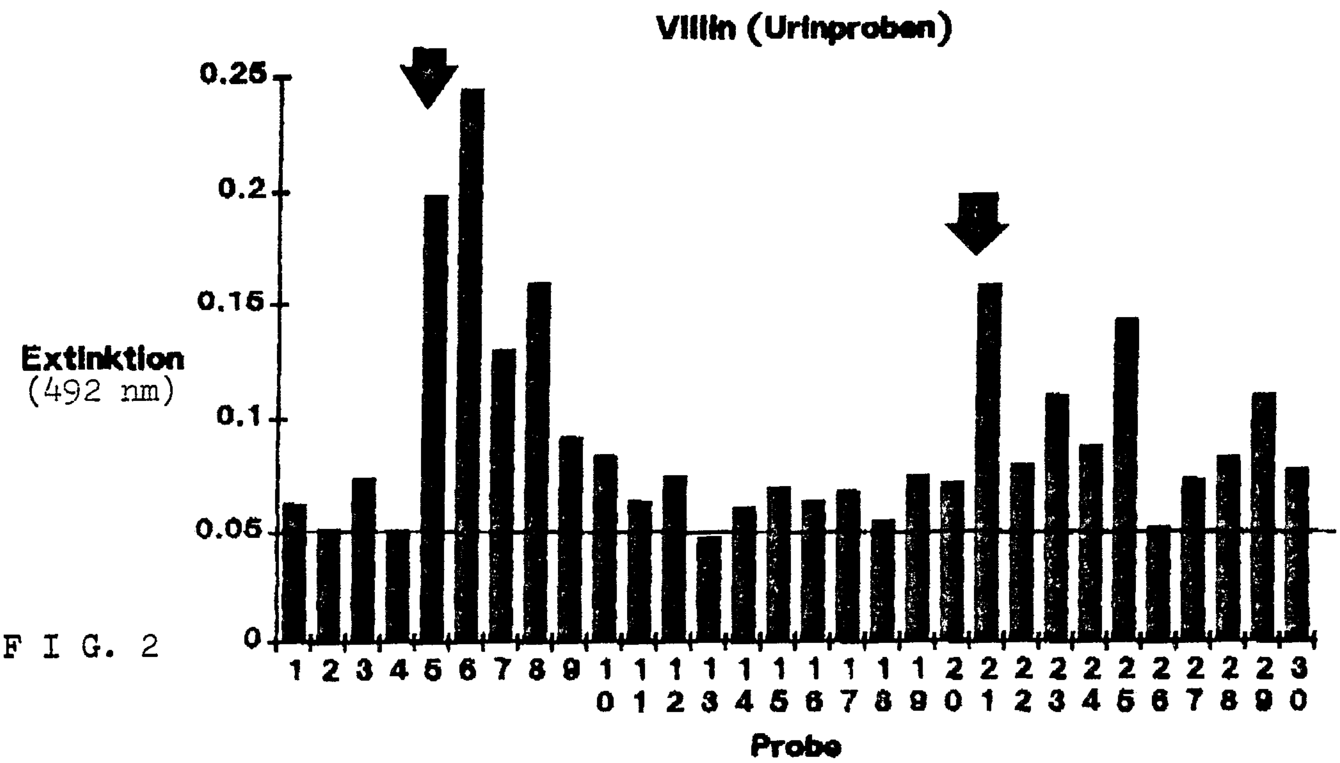

Villin (Urinproben)
Extinktion
(492 nm)
0.25
0.2
0.15
0.1
0.05
0
1 2 3 4 5 6 7 8 9 10 11 12 13 14 15 16 17 18 19 20 21 22 23 24 25 26 27 28 29 30
Probe


F I G. 2